# EUROPEAN PATENT APPLICATION

(11) **EP 2 105 087 A1**
(43) Date of publication of application: **30.09.2009**
(21) Application number: 09004475.1
(22) Date of filing: 27.03.2009
(51) Int. Cl.: A61B 1/12

(54) **Ultrasonic diagnosis system and pump apparatus**

(30) Priority: 28.03.2008 JP 2008085641; 19.05.2008 JP 2008130439
(71) Applicant: Fujifilm Corporation, Tokyo 106-0031 (JP)
(72) Inventor: Naruse, Mutsumi, Saitama-shi Saitama (JP); Yoshihara, Masatoshi, Saitama-shi Saitama (JP)
(74) Representative: Höhfeld, Jochen

(57) **Abstract**

An ultrasonic endoscope (10, 122, 150) has an ultrasonic transducer (75) for emitting and receiving ultrasonic waves, a balloon support (70), having the ultrasonic transducer, for supporting a balloon (52) mounted thereon to cover the ultrasonic transducer hermetically and removably. In an ultrasonic diagnosis system, the ultrasonic endoscope includes an inflation button (56, 124, 143) adapted to inflating the balloon. A deflation button (57, 125, 144) is adapted to deflating the balloon. Furthermore, a flow line (30, 54) extends to the balloon to pass water (24). A pump apparatus (22, 112, 117, 154) includes a rotary pump (100) for drawing the water through the flow line, to cause the water to flow into and out of the balloon. A pump controller (102) controls the rotary pump, inflates the balloon by supplying the water in response to an inflation signal from the inflation button, and deflates the balloon by discharging the water in response to a deflation signal from the deflation button.

## Description

### BACKGROUND OF THE INVENTION

### 1. Field of the Invention

The present invention relates to an ultrasonic diagnosis system and pump apparatus. More particularly, the present invention relates to an ultrasonic diagnosis system and pump apparatus in which a balloon at an end of an ultrasonic endoscope can operate reliably for inflation and deflation.

### 2. Description Related to the Prior Art

Ultrasonic diagnosis in the medical field is a non-invasive diagnosis for applying ultrasonic waves to an object in a patient's body, to observe a state of the object by receiving and imaging echo ultrasonically reflected from the object. An example of ultrasonic diagnosis is in-vivo diagnosis for applying the ultrasonic waves upon entry of a probe in a body cavity. It is possible in the in-vivo diagnosis to examine the tissue of the body cavity or gastrointestinal tract more precisely than in extracorporeal device diagnosis with the ultrasonic waves. The in-vivo diagnosis is important specifically for diagnosing the depth of ulcer, tumor and the like in the body.

Examples of instruments for the in-vivo diagnosis include an ultrasonic endoscope having an ultrasonic transducer array and CCD at its distal end, and an ultrasonic probe for use by insertion through a forceps channel of an endoscope. If there is a clearance between the ultrasonic endoscope or the ultrasonic probe and the surface in the body cavity, air exists to attenuate the ultrasonic waves remarkably. In view of such a problem, U.S.P. No. 6, 142, 945 (corresponding to JP-A 11-155865) discloses a use of a resilient balloon secured to the end of the ultrasonic endoscope of the ultrasonic probe. The balloon is filled with transmission medium such as water, inflated, and kept in tight contact with a wall in the body cavity. Then the ultrasonic waves are emitted from inside the balloon. This is effective in preventing attenuation of the ultrasonic waves with air.

U.S.P. No. 6,142,945 (corresponding to JP-A 11-155865) discloses the use of a syringe pump for inflation and deflation of the balloon. A physician or operator must use his or her first hand to steer the ultrasonic endoscope manually, and at the same time use his or her second hand to operate the syringe pump. A problem of high complexity arises in the technique disclosed in this document.

Also, it is conceivable to use an electric pump for supplying and discharging the transmission medium in relation to the balloon for the purpose of the inflation and deflation of the balloon. The second hand of the physician can be made free, because of no need of manually holding the syringe pump. However, the physician must operate a switch on a pump body which is installed separately, so that operability of the construction for operating the conduits is not higher.

If the physician carries out the inflation and deflation of the balloon, it is likely to damage the tissue surface of the body cavity with an error in operation of the switch, because he or she must gaze the switch in place of viewing a display panel displaying an ultrasonic or endoscopic image. It is conceivable to connect a foot switch with the pump for the purpose of driving and stopping the same. However, an error in operation of the foot switch is likely to occur specifically because a plurality of the foot switches are used additionally in connection with other medical instruments.

It is conceivable to carry out the inflation and deflation of the balloon by use of conduits for supply of air and water to the body cavity in the in-vivo diagnosis with the ultrasonic endoscope. However, the conduits for supply of air and water have a complicated structure in which valves change over the conduits between open and closed states. Applied use of the conduits of supply of air and water for the inflation and deflation of the balloon causes a problem of higher complexity in the structure, because the numbers of the valves and the conduits increase. The structural complexity will lower the reliability, because jamming is more likely to occur in the conduits.

### SUMMARY OF THE INVENTION

In view of the foregoing problems, an object of the present invention is to provide an ultrasonic diagnosis system and pump apparatus in which a balloon at an end of an ultrasonic endoscope can operate reliably for inflation and deflation.

In order to achieve the above and other objects and advantages of this invention, an ultrasonic diagnosis system having an ultrasonic endoscope and a pump apparatus is provided. The ultrasonic endoscope includes an ultrasonic transducer for emitting ultrasonic waves and receiving reflected ultrasonic waves. A balloon support supports a resilient balloon mounted thereon hermetically and removably to cover the ultrasonic transducer. A first flow opening passes an ultrasonic transmission medium. One flow channel is formed between the first flow opening and the balloon support, for flow of the transmission medium into and out of the balloon in forward and backward directions. An inflation input device inputs a signal to the pump apparatus for inflating the balloon. A deflation input device inputs a signal to the pump apparatus for deflating the balloon. The pump apparatus includes a second flow opening connected with the first flow opening by a tube. A pump supplies the transmission medium through the tube and the flow channel to inflate the balloon, and discharges the transmission medium from the balloon to deflate the balloon. There is a pump controller for control of the pump in response to the inflation signal to supply the transmission medium, and for control of the pump in response to the deflation signal to discharge the transmission medium.

Furthermore, a processor is connected with the ultrasonic endoscope and the pump apparatus, for inputting the inflation and deflation signals to the pump apparatus.

The ultrasonic endoscope includes plural switches adapted to selected functions, and including a first switch for constituting the inflation input device and a second switch for constituting the deflation input device.

The ultrasonic endoscope includes a transmitter, having the inflation and deflation input devices, for wirelessly transmitting the inflation and deflation signals.

Furthermore, a retention mechanism retains the transmitter on the ultrasonic endoscope removably.

The pump apparatus further includes a first flow setting device for setting a supply amount of supply of the transmission medium. A second flow setting device sets a discharge amount of discharge of the transmission medium. The controller controls the pump to supply the transmission medium at the supply amount and to discharge the transmission medium at the discharge amount.

The pump apparatus further includes a measuring device for measuring at least one of a supply amount of the transmission medium to the balloon and a discharge amount of the transmission medium from the balloon.

Furthermore, an evaluator evaluates the supply amount or the discharge amount by comparison with a reference amount. An alarm unit generates an alarm signal if the supply amount or the discharge amount has become higher than the reference amount.

In one preferred embodiment, the measuring device includes a timer for measuring at least one of a supply time and discharge time of the transmission medium with the pump.

In another preferred embodiment, the pump is a rotary pump for supplying and discharging the transmission medium by rotations. The measuring device detects at least one of a number and angle of the rotations of the rotary pump.

In still another preferred embodiment, the measuring device is a flow meter.

The balloon support includes first and second ring portions positioned at respectively first and second ends of the balloon. A first coupling portion is disposed in an endoscope distal end portion and close to the ultrasonic transducer, and having the first ring portion fitted thereon. A second coupling portion is disposed between the ultrasonic transducer and an endoscope proximal side, and having the second ring portion fitted thereon.

In one aspect of the invention, a pump apparatus for use in connection with an ultrasonic endoscope is provided. The ultrasonic endoscope includes an ultrasonic transducer for emitting and receiving ultrasonic waves, a balloon support, having the ultrasonic transducer, for supporting a balloon mounted thereon to cover the ultrasonic transducer hermetically and removably, an inflation input device adapted to inflating the balloon, and a deflation input device adapted to deflating the balloon. In the pump apparatus, a pump draws transmission medium through a flow line disposed to extend to the balloon, to cause the transmission medium to flow into and out of the balloon. A pump controller controls the pump, inflates the balloon by supplying the transmission medium in response to an inflation signal from the inflation input device, and deflates the balloon by discharging the transmission medium in response to a deflation signal from the deflation input device.

In another aspect of the invention, an ultrasonic diagnosis system is provided, and includes an ultrasonic endoscope having an ultrasonic transducer for emitting and receiving ultrasonic waves, a balloon support, having the ultrasonic transducer, for supporting a balloon mounted thereon to cover the ultrasonic transducer hermetically and removably. In the ultrasonic diagnosis system, the ultrasonic endoscope includes an inflation input device adapted to inflating the balloon. A deflation input device is adapted to deflating the balloon. Furthermore, a flow line extends to the balloon to pass transmission medium. A pump apparatus includes a pump for drawing the transmission medium through the flow line, to cause the transmission medium to flow into and out of the balloon. A pump controller controls the pump, inflates the balloon by supplying the transmission medium in response to an inflation signal from the inflation input device, and deflates the balloon by discharging the transmission medium in response to a deflation signal from the deflation input device.

Accordingly, the balloon at an end of an ultrasonic endoscope can operate reliably for inflation and deflation, because the inflation and deflation input devices are effective in easily instructing the flow of the transmission medium into or out of the balloon.

### BRIEF DESCRIPTION OF THE DRAWINGS

The above objects and advantages of the present invention will become more apparent from the following detailed description when read in connection with the accompanying drawings, in which:
Fig. 1 is a perspective view illustrating an ultrasonic diagnosis system;
Fig. 2A is a vertical section illustrating a distal end of an ultrasonic endoscope where a balloon is separated;
Fig. 2B is a vertical section illustrating the same as Fig. 2A but where the balloon is secured;
Fig. 3 is a block diagram schematically illustrating the ultrasonic diagnosis system;
Fig. 4A is a front elevation illustrating appearance of a display panel with a first alarm message;
Fig. 4B is a front elevation illustrating appearance of a display panel with a second alarm message;
Fig. 5 is a block diagram schematically illustrating one preferred ultrasonic diagnosis system in which supply and discharge amounts are measured;
Fig. 6 is a block diagram schematically illustrating another preferred ultrasonic diagnosis system having a rotary encoder;
Fig. 7 is a perspective view illustrating one preferred embodiment having plural buttons;
Fig. 8 is a block diagram schematically illustrating an ultrasonic diagnosis system having the plural buttons;
Fig. 9A is a table illustrating a set of button functions stored in a button function memory;
Fig. 9B is a table illustrating another preferred set of button functions;
Fig. 10 is a perspective view illustrating one preferred embodiment having a remote control unit;
Fig. 11 is a block diagram schematically illustrating an ultrasonic diagnosis system having the remote control unit.

### DETAILED DESCRIPTION OF THE PREFERRED EMBODIMENT(S) OF THE PRESENT INVENTION

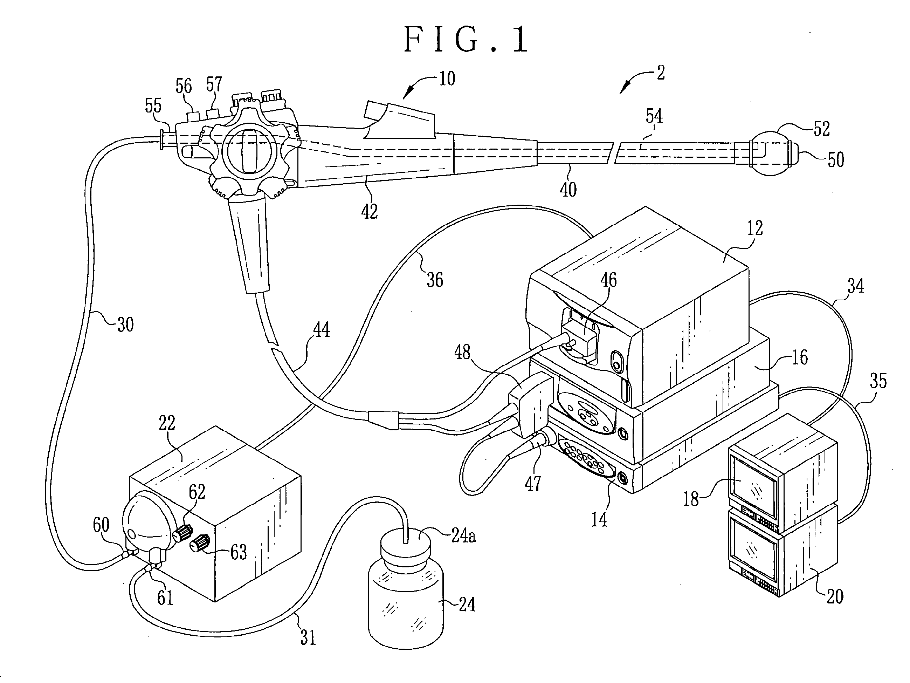

In Fig. 1, an ultrasonic diagnosis system 2 includes an ultrasonic endoscope 10, an ultrasonic processor 12, an endoscope processor 14, a light source unit 16, an ultrasonic monitor display panel 18, an endoscope monitor display panel 20, a pump apparatus 22, and a water tank 24 as water source. The ultrasonic endoscope 10 picks up an image of an object in a body cavity or gastrointestinal tract. The ultrasonic processor 12 creates an ultrasonic image. The endoscope processor 14 creates an endoscopic image. The light source unit 16 generates illumination light for illuminating the object. The ultrasonic monitor display panel 18 displays the ultrasonic image. The endoscope monitor display panel 20 displays the endoscopic image. The pump apparatus 22 causes water to flow into and out of the ultrasonic endoscope 10. The water tank 24 stores the water in connection with the pump apparatus 22.

The ultrasonic endoscope 10 includes an insertion tube 40, a handle 42 and a universal cable 44. The handle 42 is a base portion from which the insertion tube 40 extends. A first end of the universal cable 44 is connected with the handle 42. A second end of the universal cable 44 has a contact plug 46 or connector, an endoscope plug 47 or connector, and a light source plug 48 or connector. The contact plug 46 is connected with the ultrasonic processor 12. The endoscope plug 47 is connected with the endoscope processor 14. The light source plug 48 is connected with the light source unit 16. Thus, the ultrasonic endoscope 10 is connected with the ultrasonic processor 12, the endoscope processor 14 and the light source unit 16 by the plugs 46-48.

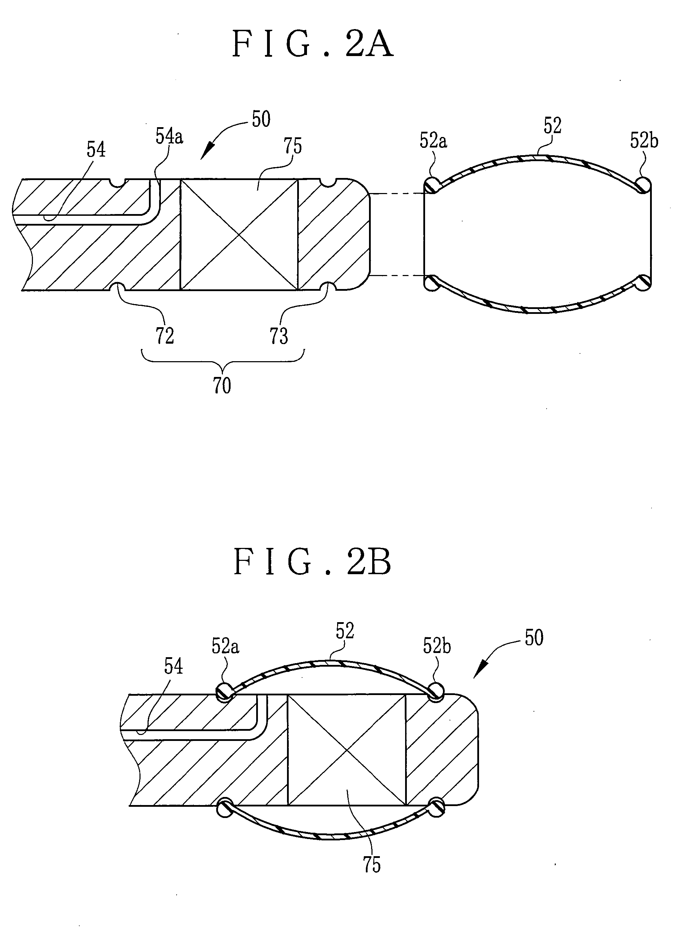

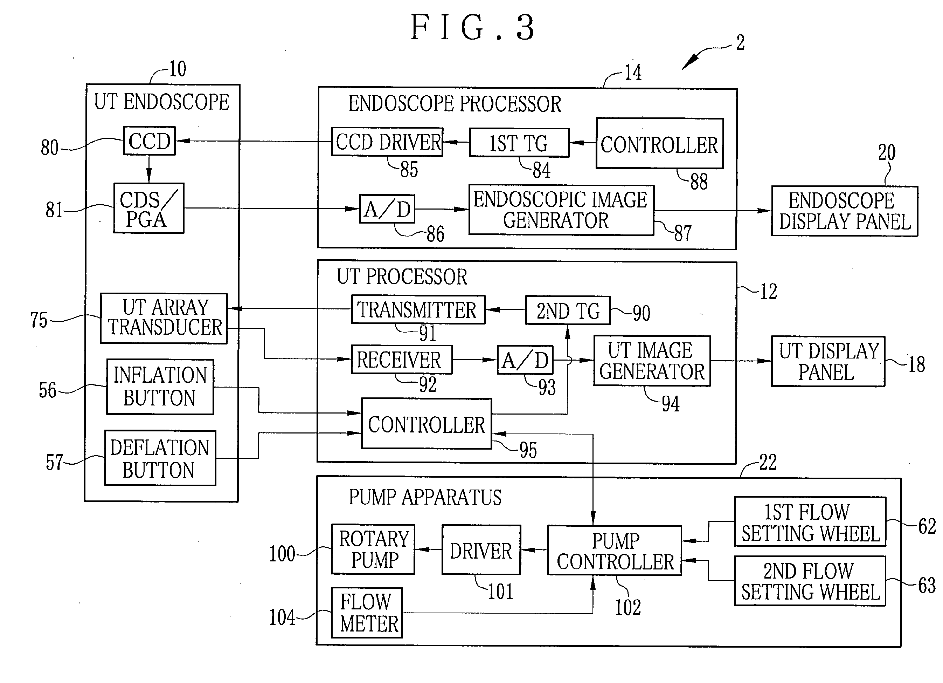

The insertion tube 40 is in a tubular form, and has flexibility. A head assembly 50 at a distal end of the insertion tube 40 has an ultrasonic array transducer 75 of Fig. 2 and a CCD 80 of Fig. 3. A balloon 52 with resiliency is attached to the head assembly 50. The balloon 52 is entered in the body cavity in a deflated state in tight contact with the outer surface of the head assembly 50. For the time of emitting ultrasonic waves from the ultrasonic transducer 75, the balloon 52 is inflated by water supplied by the pump apparatus 22. The contact of the head assembly 50 with the object in the body cavity is tightened at the balloon 52. Ultrasonic waves from the ultrasonic transducer 75 and reflected ultrasonic waves are prevented from attenuation with air or other fluid. After the inflation, the balloon 52 is deflated again by draining water. An example of the material for the balloon 52 is latex rubber. Note that the water for the balloon 52 is preferably degassed water obtained by degassing of dissolved gas.

A flow channel 54 is formed through the insertion tube 40 and the handle 42 for flow of water to and from the balloon 52. A first open end of the flow channel 54 appears in the head assembly 50. A flow opening 55 is disposed at the proximal end of the handle 42. A second open end of the flow channel 54 is open through the flow opening 55. The handle 42 includes an inflation button 56 and a deflation button 57 as input devices. The inflation button 56 is operable to cause the pump apparatus 22 to dispense water to inflate the balloon 52. The deflation button 57 is operable to cause the pump apparatus 22 to discharge water to deflate the balloon 52.

A first flow opening 60 and a second flow opening 61 are formed in the pump apparatus 22. Tubes 30 and 31 with flexibility are connected with respectively the first and second flow openings 60 and 61. A first tube end of the tube 30 is connected with the first flow opening 60. A second tube end of the tube 30 is connected with the flow opening 55. A first end of the tube 31 is connected with the second flow opening 61. A second end of the tube 31 is connected with a tube connector 24a of the water tank 24. Thus, a flow line from the water tank 24, via the tube 31, the pump apparatus 22, the tube 30 and the flow channel 54 to the balloon 52 is defined.

A rotary pump 100 of Fig. 3 is included in the pump apparatus 22, makes a rotor to rotate for creating a flow of ultrasonic transmission medium or water in the flow line, and changes over a direction of the flow selectively by changing the rotational direction of the rotor. The pump apparatus 22 drives the rotary pump 100 to draw transmission medium from one of the first and second flow openings 60 and 61 and deliver transmission medium through a remaining one of those. Note that JP-A 2001-321329 discloses details of the construction of the rotary pump 100.

When the inflation button 56 is depressed, the rotary pump 100 in the pump apparatus 22 is controlled for suction through the second flow opening 61 and delivery through the first flow opening 60, to draw water from the water tank 24 into the balloon 52. When the deflation button 57 is depressed, the rotary pump 100 is controlled for suction through the first flow opening 60 and delivery through the second flow opening 61, to draw water from the balloon 52 back to the water tank 24.

The pump apparatus 22 includes a first flow setting wheel 62 and a second flow setting wheel 63 as flow setting device. The first flow setting wheel 62 adjusts a flow volume in the supply of water per unit time, namely a flow rate. The second flow setting wheel 63 adjusts a flow volume in the discharge of water per unit time, namely a flow rate. The pump apparatus 22 discretely controls the flow volumes of the supply and discharge according to the settings of the flow setting wheels 62 and 63. This is effective in decreasing the flow volume for the supply and increasing the flow volume for the discharge. It is possible to prevent the balloon 52 from inflating abruptly or breaking, and keep the balloon 52 tightly secured to the head assembly 50 without drop. The balloon 52 can be deflated rapidly to raise the efficiency in the diagnosis.

A cable 34 connects the ultrasonic processor 12 to the ultrasonic monitor display panel 18. The ultrasonic processor 12 outputs an ultrasonic image through the cable 34 for the ultrasonic monitor display panel 18 to display the same. A cable 35 connects the endoscope processor 14 to the endoscope monitor display panel 20. The endoscope processor 14 outputs the endoscopic image through the cable 35 for the endoscope monitor display panel 20 to display the same. A cable 36 connects the ultrasonic processor 12 to the pump apparatus 22. Various signals are transmitted and received between the ultrasonic processor 12 and the pump apparatus 22 through the cable 36.

In Fig. 2A, the balloon 52 has a shape of a drum, or a shape of a rotating body with a greater diameter at an intermediate portion. Ring portions 52a and 52b project at ends of the balloon 52. Diameters of the ring portions 52a and 52b are slightly smaller than a diameter of the head assembly 50.

A balloon support 70 is disposed at the head assembly 50 of the insertion tube 40 of the ultrasonic endoscope 10, and includes coupling grooves 72 and 73. The coupling grooves 72 and 73 extend circumferentially and are suitable for the ring portions 52a and 52b of the balloon 52. An interval between the coupling grooves 72 and 73 is substantially equal to that between the ring portions 52a and 52b. To secure the balloon 52 to the balloon support 70, at first the ring portions 52a and 52b are spread radially, before the head assembly 50 is inserted in the balloon 52. The ring portions 52a and 52b are fitted in the coupling grooves 72 and 73 with resiliency of the balloon 52. In Fig. 2B, the balloon 52 is secured to the balloon support 70 removably and hermetically.

An end opening 54a is open between the coupling grooves 72 and 73 as an end opening of the flow channel 54. The balloon support 70 is connected with the flow opening 55 by the end opening 54a, where water is drawn into and out of the balloon 52. The ultrasonic transducer 75 is disposed between the coupling grooves 72 and 73 for emitting ultrasonic waves and receiving echo or reflected ultrasonic waves. The ultrasonic transducer 75 is covered by the balloon 52 attached to the balloon support 70. Thus, ultrasonic waves are applied through the inside of the balloon 52, and can be prevented from attenuation with air.

In Fig. 3, circuit arrangement of the ultrasonic diagnosis system 2 is schematically illustrated. The ultrasonic endoscope 10 includes a correlated double sampling/programmable gain amplifier (CDS/PGA) 81 and the CCD 80 in addition to the inflation and deflation buttons 56 and 57 and the ultrasonic transducer 75. The CCD 80 and the ultrasonic transducer 75 are disposed in the head assembly 50 at the end of the insertion tube 40. The CCD 80 picks up an object image of object light incident through the imaging window, to output an image signal according to the object image. The CDS/PGA 81 processes the image signal from the CCD 80 in the noise reduction and amplification.

The ultrasonic transducer 75 is an array of plural ultrasonic transducer elements. The ultrasonic transducer 75 emits ultrasonic waves. The ultrasonic transducer 75 receives ultrasonic waves reflected by an object in a body cavity, converts those piezoelectrically, and produces a detection signal according to the reflected ultrasonic waves.

The endoscope processor 14 includes a first timing generator 84, a CCD driver 85, an A/D converter 86, an endoscopic image generator 87 and a controller 88 for controlling various circuit elements in the endoscope processor 14.

The first timing generator 84 is controlled by the controller 88 and inputs a timing signal or clock pulse to the CCD driver 85. The CCD driver 85 in response to this inputs a drive signal to the CCD 80, of which the timing of reading the stored charge and an electronic shutter speed are controlled.

The A/D converter 86 converts an image signal of an analog form from the CDS/PGA 81 into digital image data. The endoscopic image generator 87 processes the digital image data in image processing of various functions, to create an endoscopic image. Also, the endoscopic image generator 87 converts the endoscopic image into a video signal (component signal, composite signal and the like) suitable for the format of the endoscope monitor display panel 20, and outputs the video signal. Thus, the endoscopic image is displayed on the endoscope monitor display panel 20.

The ultrasonic processor 12 includes a second timing generator 90, a transmitter 91, a receiver 92, an A/D converter 93, an ultrasonic image generator 94, and a controller 95. The inflation and deflation buttons 56 and 57 are connected with the controller 95 by the universal cable 44. The controller 95 is connected with the pump apparatus 22 by the cable 36.

The second timing generator 90 is controlled by the controller 95 and sends a drive pulse to the transmitter 91. The transmitter 91 in response to this supplies an excitation pulse or pulse voltage to the ultrasonic transducer 75 to emit ultrasonic waves. A detection signal is output by the ultrasonic transducer 75 upon the excitation pulse, and is received by the receiver 92, and is sent to the A/D converter 93.

The A/D converter 93 converts the detection signal of an analog form from the receiver 92 into image data of a digital form. The ultrasonic image generator 94 processes the image data from the A/D converter 93 in image processing of various functions, and creates an ultrasonic image. The ultrasonic image generator 94 converts the ultrasonic image into a video signal (component signal, composite signal or the like) according to the format of the ultrasonic monitor display panel 18, and outputs the video signal. Thus, the ultrasonic monitor display panel 18 displays the ultrasonic image.

The pump apparatus 22 includes the rotary pump 100, a driver 101 and a pump controller 102 as evaluator. The rotary pump 100 supplies water to and discharges water from the balloon 52. The driver 101 drives the rotary pump 100. The pump controller 102 controls various circuit elements of the pump apparatus 22. The flow setting wheels 62 and 63 are connected with the pump controller 102 for setting reference amounts of ultrasonic transmission medium. The pump controller 102 is connected with the controller 95 of the ultrasonic processor 12 by the cable 36.

The controller 95 of the ultrasonic processor 12 responds to depression of the inflation button 56, and sends an inflation signal to the pump controller 102 for instructing the inflation of the balloon 52. The pump controller 102 in response to the inflation signal sends a supply signal to the driver 101 according to a setting of the first flow setting wheel 62. The driver 101 controls a rotational direction and rotational speed of a rotor according to the supply signal in the rotary pump 100, and draws water from the water tank 24 to inflate the balloon 52.

When the deflation button 57 is depressed, the controller 95 sends a deflation signal to the pump controller 102 for deflation of the balloon 52. The pump controller 102 in response to this sends a discharge signal (control signal) to the driver 101 according to the reference amount set by the second flow setting wheel 63. The driver 101 drives the rotary pump 100 according to the discharge signal, and deflates the balloon 52 by discharging water.

A flow meter 104 as measuring device is also included in the pump apparatus 22 and measures a supply amount and discharge amount of water to the balloon 52 by means of the rotary pump 100. The flow meter 104 inputs measured information of the supply amount and discharge amount to the pump controller 102.

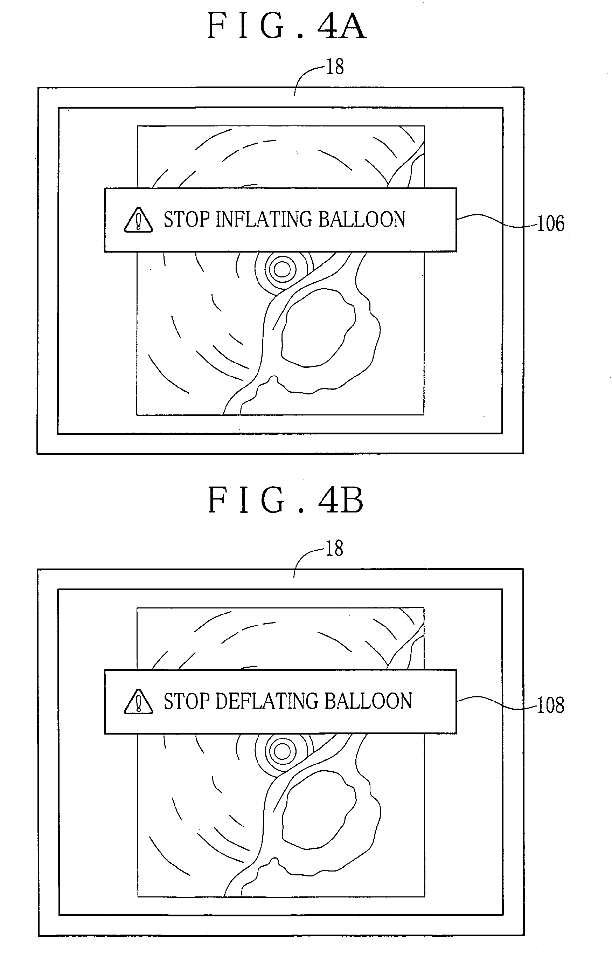

The pump controller 102, upon receiving the inflation signal for inflation, sends a supply signal to the driver 101, and checks whether the supply amount is higher than a reference amount predetermined in the system. The balloon 52, if inflated excessively, is likely to break in the body cavity or drop from the balloon support 70. Thus, the pump controller 102, if the supply amount is found higher than the reference amount, sends an inflation termination signal to the controller 95. The controller 95, upon receiving the inflation termination signal, causes the ultrasonic monitor display panel 18 to display a first alarm message 106 or dialog box as alarm device as illustrated in Fig. 4A, to indicate an alarm state for possibility of excessive inflation of the balloon 52.

The pump controller 102, when the supply amount is found more than the reference amount, sends the inflation termination signal to the controller 95 until the stop of the inflation signal from the controller 95, or until the release of depression of the inflation button 56. Display of the first alarm message 106 is continued. Thus, it is possible to prevent excessive inflation of the balloon 52 by displaying the warning.

The pump controller 102, in response to a deflation signal for deflation, sends a discharge signal to the driver 101, and checks whether the discharge amount from the flow meter 104 has become higher than a reference amount. The balloon 52, if deflated excessively, may be deformed by force of suction, or may be trapped or jammed in the end opening 54a of the flow channel 54. Also, it is likely that air or fluid enters the flow channel 54 through clearance between the ring portions 52a and 52b and the coupling grooves 72 and 73. A successive operation of inflating the balloon 52 may create bubbles by entry of the air or fluid in the balloon 52.

The pump controller 102, upon detecting that the discharge amount becomes higher than the reference amount, sends a deflation termination signal to the controller 95 to stop the deflation. The controller 95 in response to this causes a second alarm message 108 or dialog box as alarm device to appear on the ultrasonic monitor display panel 18, so as to emit an alarm signal of possibility of excessive deflation of the balloon 52. See Fig. 4B.

The pump controller 102 continues displaying the second alarm message 108 by transmission of the deflation termination signal to the controller 95 after detection of the discharge amount higher than the reference amount and until the stop of the deflation signal from the controller 95, namely until release of depression of the deflation button 57. Thus, excessive deflation of the balloon 52 can be prevented by continuing indication of the second alarm message 108. Note that the reference amounts for use in the evaluation may be predetermined suitably for the volume of the balloon 52 or other information.

The operation of the ultrasonic diagnosis system 2 is described now. At first, a physician or operator in a hospital sets up various elements of the ultrasonic diagnosis system 2 as illustrated in Fig. 1. The balloon 52 is completely emptied of water, and is retained in a deflated state to contact the outer surface of the head assembly 50 tightly. The physician depresses a start button (not shown) of the endoscope processor 14. A signal for start of the diagnosis is sent to various circuit elements in the ultrasonic diagnosis system 2.

The controller 88 of the endoscope processor 14, in response to the command signal for the start, controls the first timing generator 84 to start driving the CCD 80 with the CCD driver 85. The CCD 80 is caused by the CCD driver 85 to pick up an object image of object light incident through the imaging window, to output an image signal according to the object image. The image signal is processed by the CDS/PGA 81 for noise reduction and amplification, output to the A/D converter 86, and converted into image data of a digital form. The image data is input to the endoscopic image generator 87. The endoscopic image generator 87 processes the image data in the image processing of various functions, to create an endoscopic image. The endoscopic image generator 87 converts the endoscopic image into a video signal suitable for the format of the endoscope monitor display panel 20, and outputs the video signal. Thus, the endoscopic image is displayed on the endoscope monitor display panel 20.

The controller 95 in the ultrasonic processor 12 starts the control of the second timing generator 90 upon receiving the start signal. The second timing generator 90 is controlled by the controller 95 to input a drive pulse to the transmitter 91. In response to the drive pulse, the transmitter 91 supplies an excitation pulse to the ultrasonic array transducer 75.

The ultrasonic transducer 75 emits ultrasonic waves according to the excitation pulse from the transmitter 91, and receives ultrasonic waves reflected by the object. The reflected ultrasonic waves are converted piezoelectrically to create a detection signal. The detection signal is transmitted to the receiver 92 in the ultrasonic processor 12. The receiver 92 outputs the detection signal to the A/D converter 93.

The detection signal is converted into image data of a digital form by the A/D converter 93, and input to the ultrasonic image generator 94. The ultrasonic image generator 94 creates an ultrasonic image by image processing of the digital image data. The ultrasonic image is converted into a video signal compatible to the ultrasonic monitor display panel 18, and outputs the video signal. Thus, the ultrasonic monitor display panel 18 displays the ultrasonic image.

When each of the display panels 18 and 20 is started for display, a physician enters the insertion tube 40 of the ultrasonic endoscope 10 in a body cavity of a patient's body for imaging of an object. At first, an endoscopic image is viewed. If a lesion is discovered in the object in the endoscopic image, or if he or she wishes to view the tissue of the object more precisely, then observation is changed over to an ultrasonic image.

For imaging of the ultrasonic image, the inflation button 56 is depressed to inflate the balloon 52 by supplying water from the water tank 24. A supply amount or flow volume of the supply to the balloon 52 is adjustable by operation of the first flow setting wheel 62. Upon the depression of the inflation button 56, the pump controller 102 checks whether the supply amount to the balloon 52 has become higher than a reference amount.

The pump controller 102, if the supply amount is found higher than the reference amount, transmits an inflation termination signal to the controller 95. The controller 95 in response to this causes the ultrasonic monitor display panel 18 to display the first alarm message 106 to inform possibility of excessive inflation of the balloon 52. Thus, excessive inflation of the balloon 52 can be prevented.

The physician causes the balloon 52 after the inflation to contact an object or lesion tightly. Then an ultrasonic image of the object is displayed on the ultrasonic monitor display panel 18. A state of submucosal tissue of the object can be viewed in detail.

The physician moves and separates the balloon 52 from the object upon he or she finishes viewing the ultrasonic image. The deflation button 57 is depressed to deflate the balloon 52 by discharging water. It is possible by operating the second flow setting wheel 63 to determine a flow volume of the water of discharge from the balloon 52. As the flow volumes in the course of the supply and discharge are discretely determined, adjusted operation is possible. For example, the balloon 52 can be inflated slowly with a small flow volume (flow rate) in the supply, and can be deflated quickly with a great flow volume (flow rate) in the discharge.

When the deflation button 57 is depressed, the pump controller 102 checks whether the discharge amount of the balloon 52 has become higher than a reference amount. If the discharge amount is found higher than the reference amount, then the pump controller 102 outputs a deflation termination signal to the controller 95 to stop the deflation. The controller 95, upon receiving the deflation termination signal, causes the ultrasonic monitor display panel 18 to display the second alarm message 108 as alarm signal of possibility of excessive deflation of the balloon 52. This is effective in preventing the excessive deflation of the balloon 52.

Therefore, operability of the ultrasonic diagnosis system 2 can be high because the balloon 52 can be inflated and deflated by manual operation of the inflation and deflation buttons 56 and 57 on the handle 42 of the ultrasonic endoscope 10. Also, an operator can carefully observe the display panels 18 and 20, and can be free from errors in operation of other medical instruments. Safety in the course of the diagnosis can be high. Furthermore, high reliability of the ultrasonic diagnosis system 2 can be obtained, because the flow channel 54 as single conduit can operate for flow in forward and backward directions without need of complicated structure for supply and discharge of water with the balloon 52.

In the above embodiment, the ultrasonic processor 12 is a control unit for inputting inflation and deflation signals to the pump apparatus 22. However, the endoscope processor 14 can be a control unit for inputting inflation and deflation signals to the pump apparatus 22. Also, the inflation and deflation buttons 56 and 57 may be connected with the pump controller 102 of the pump apparatus 22 to input the signals to the pump apparatus 22 directly from the ultrasonic endoscope 10.

In the above embodiment, the inflation and deflation buttons 56 and 57 are used. However, structures for control of inflation and deflation may have any suitable form other than the inflation and deflation buttons 56 and 57.

In the above embodiment, the flow meter 104 measures the flow volumes of the supply and discharge of water with the balloon 52. Furthermore, a pressure meter is preferably associated with the pump apparatus 22 to measure the flow volumes of the supply and discharge of water according to pressure applied in the conduit of the water.

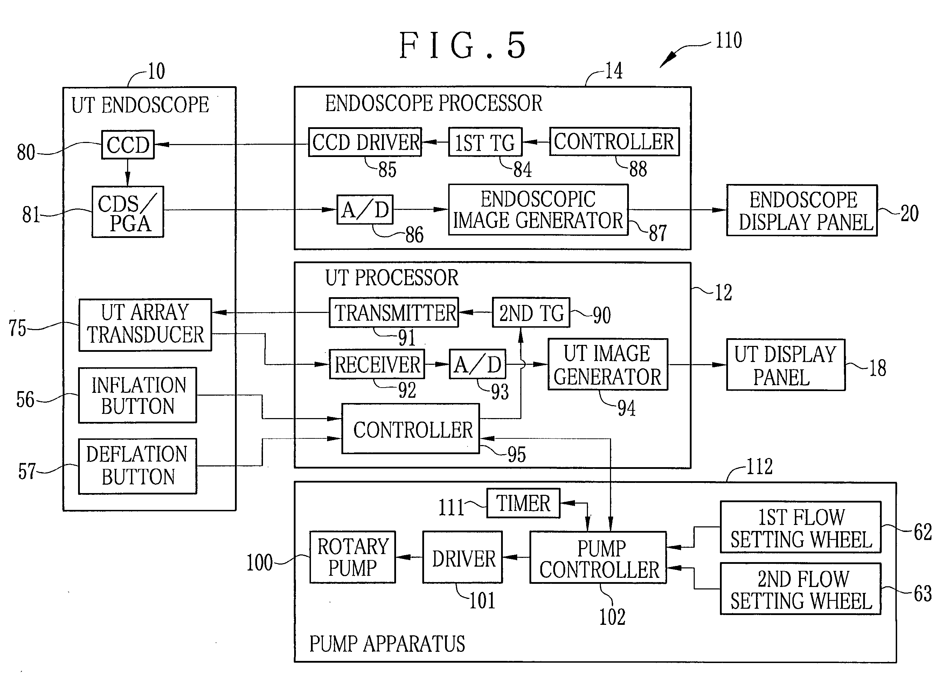

In Fig. 5, one preferred ultrasonic diagnosis system 110 is illustrated, in which a pump apparatus 112 has a timer 111 as measuring device for measuring at least one of the supply time or discharge time in operation of the rotary pump 100.

The timer 111 is connected with the pump controller 102. The timer 111 measures a length of a period of a supply signal or discharge signal from the pump controller 102 to the driver 101. The pump controller 102 stores a data table (not shown) of a relationship between the length of the period of the supply signal or discharge signal and the supply amount or discharge amount, the relationship being determined for respective values of adjustment of the flow setting wheels 62 and 63. The pump controller 102 determines the supply amount or discharge amount according to the data table and time measured by the timer 111, and carries out the evaluation described above.

It is possible to measure time of continuation of the inflation or deflation signal from the controller 95 of the ultrasonic processor 12 to the pump controller 102 by way of supply time or discharge time. Furthermore, it is possible to convert the reference amounts of the supply amount or discharge amount into supply time or discharge time, and to evaluate the supply time or discharge time directly obtained by the timer 111, for the purpose of the above evaluation. This is effective in place of obtaining the supply amount or discharge amount in the pump controller 102.

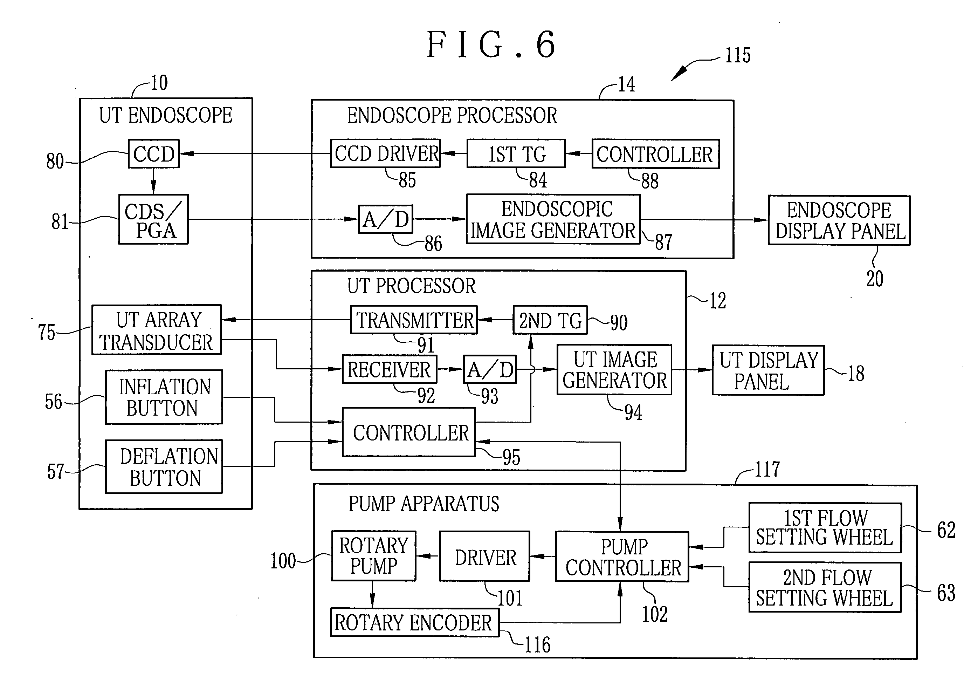

In Fig. 6, another preferred ultrasonic diagnosis system 115 is illustrated. A pump apparatus 117 includes a rotary encoder 116 as detector or measuring device for detecting at least one of the number of rotations of the rotor and its rotational angle. In a manner similar to the example of Fig. 5, the pump controller 102 determines the supply amount or discharge amount according to the data table and a detection result from the rotary encoder 116, the data table being constituted by the relationship between the rotation number or rotational angle of the rotor and the supply amount or discharge amount, the relationship being determined for respective settings of the flow setting wheels 62 and 63. It is also possible to carry out the evaluation according to the rotation number or rotational angle itself of the rotor detected by the rotary encoder 116.

The embodiments of Figs. 5 and 6 have advantages in reducing the cost in comparison with the use of the flow meter or pressure meter. It is possible to measure the supply amount or discharge amount with a simple structure without complicated structure to detect a flow volume or pressure. Should a flow meter or pressure meter be used, there is a problem of additional operation of cleaning and disinfection as water directly contacts the meter. However, it is possible according to the embodiments to measure the supply amount or discharge amount without contacting the water. There is no problem of additional cleaning and disinfection.

If the inflation or deflation button 56 or 57 is depressed continually, then the pump controller 102 obtains an accumulated value of the supply amount or discharge amount. If a first button among those and identical with that depressed previously is depressed, then the supply amount or discharge amount of this time is added to an accumulated value of the supply amount or discharge amount. If a second button different from the first depressed previously is depressed, then the supply amount or discharge amount of this time is subtracted from an accumulated value of the supply amount or discharge amount. Thus, it is possible exactly to recognize the total of the water supplied into the balloon 52.

In the embodiment, the first and second alarm messages 106 and 108 operate for the alarm information. Information displayed on the ultrasonic monitor display panel 18 is the alarm information. However, an alarm device may be an alarm sound source, alarm light source and the like for alarm information related to an increase in the supply amount and discharge amount over the reference amount. In the above embodiment, the alarm device operates upon an increase of the supply amount and discharge amount over the reference amount. However, it is possible to stop driving the rotary pump 100 in response to detection of the supply and discharge amounts coming over the reference amount.

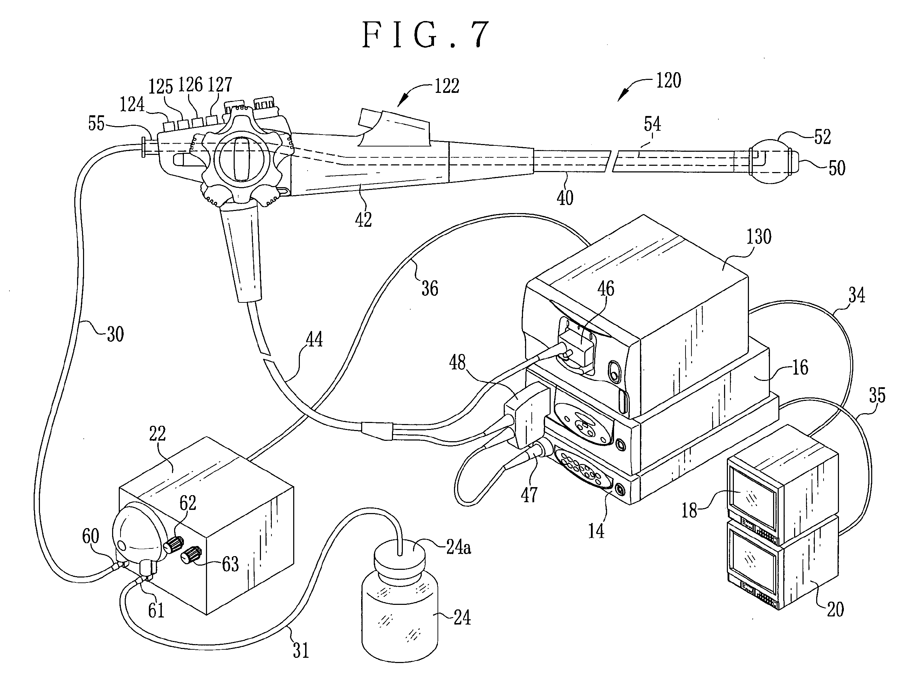

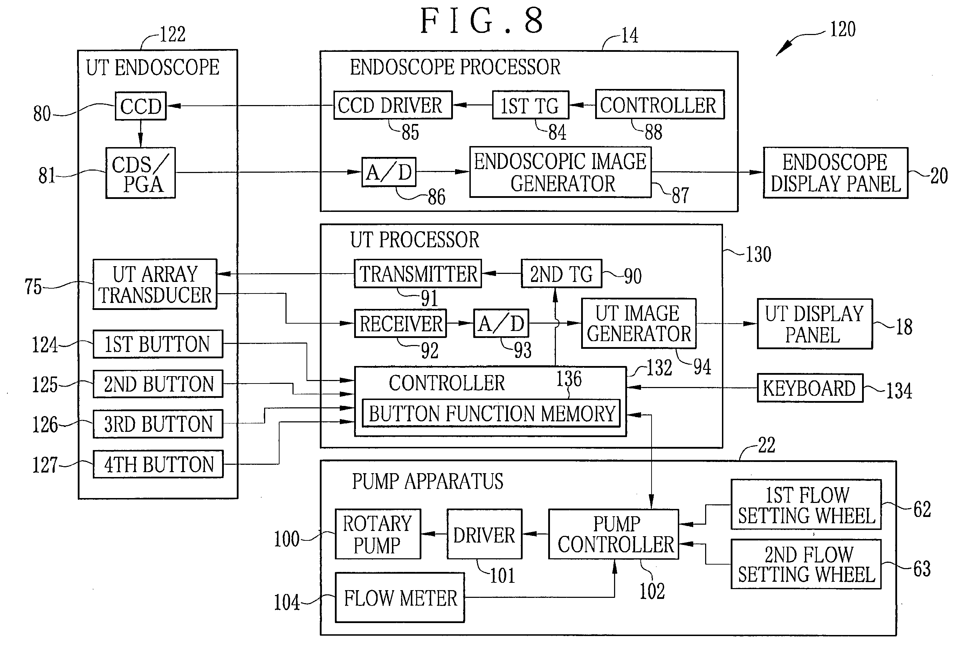

One preferred embodiment of the invention is described now. Elements similar to those of the above embodiment are designated with identical reference numerals. In Fig. 7, an ultrasonic endoscope 122 in an ultrasonic diagnosis system 120 has a first button 124, a second button 125, a third button 126 and a fourth button 127 disposed on the handle 42 as pushbuttons or switches. In Fig. 8, signal lines from the first to fourth buttons 124-127 extend to a controller 132 in an ultrasonic processor 130 by use of the universal cable 44 or the like.

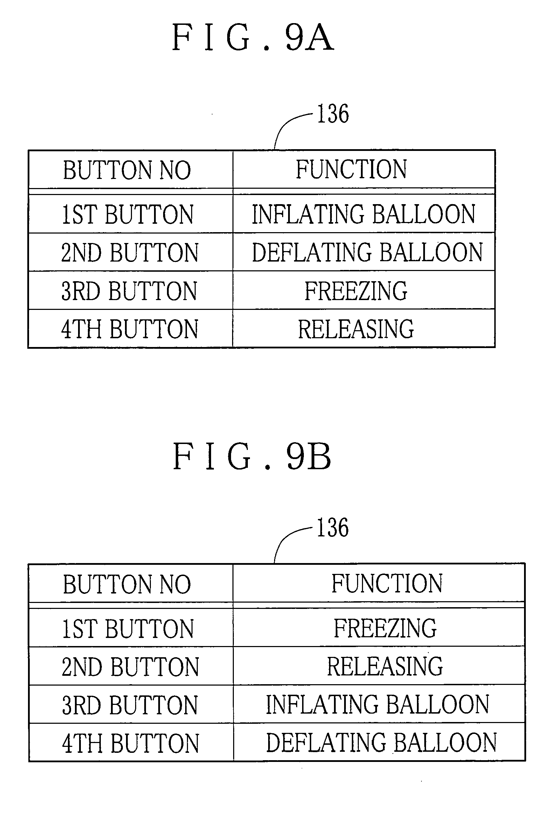

A keyboard 134 is associated with the controller 132 for inputting alphanumeric information, command signals and other various signals. A button function memory 136 is incorporated in the controller 132 and stores functions of the first to fourth buttons 124-127. In Fig. 9, data in the button function memory 136 are relationships between information of the first to fourth buttons 124-127 and the functions assigned to those. When the first to fourth buttons 124-127 are depressed, the controller 132 refers to the button function memory 136 to check the functions, so as to perform tasks according to the functions.

In Fig. 9A, the first button 124 is assigned with a function of inflation of the balloon. The first button 124 operates by way of the inflation button 56 of the first embodiment. When the first button 124 is depressed, the controller 132 sends an inflation signal to inflate the balloon 52. The second button 125 is assigned with a function of deflation of the balloon. The second button 125 operates by way of the deflation button 57 of the first embodiment. When the second button 125 is depressed, the controller 132 generates a deflation signal to deflate the balloon 52.

The third button 126 is assigned with a freezing function, to operate as a freezing button. When the third button 126 is depressed, the controller 132 causes the display panels 18 and 20 to display ultrasonic and endoscopic images in a form of still images. The fourth button 127 is assigned with a release function, to operate as a release button. When the fourth button 127 is depressed, the controller 132 operates for recording of ultrasonic and endoscopic images in a form of still images.

Functions of the first to fourth buttons 124-127 can be set up as desired by manual operation of the keyboard 134 to rewrite the button function memory 136. For example, when data are rewritten in the button function memory 136 from the state of Fig. 9A to the state of Fig. 9B, the first button 124 comes to operate as a freezing button in place of operation of the inflation button 56. The second button 125 comes to operate as a release button in place of operation of the deflation button 57. The third button 126 comes to operate as the inflation button 56 in place of operation of the freezing button. The fourth button 127 comes to operate as the deflation button 57 in place of operation of the release button.

The first to fourth buttons 124-127 can be set for desired functions. The working efficiency of the ultrasonic endoscope 122 can be high because the inflation and deflation buttons 56 and 57 can be positioned by use of the first to fourth buttons 124-127 according to a specific diagnosis or preferences in the department of physicians.

In the above embodiment, the button function memory 136 is incorporated in the controller 132. However, the button function memory 136 may be incorporated in the ultrasonic endoscope 122, where the functions of the first to fourth buttons 124-127 may be discerned in the ultrasonic endoscope 122. The positions, number and functions of plural buttons in the handle 42 are not limited to the above embodiment. Also, switches in the handle 42 are not limited to the first to fourth buttons 124-127 as depressible switches, but can be slide switches, toggle switches and other switches.

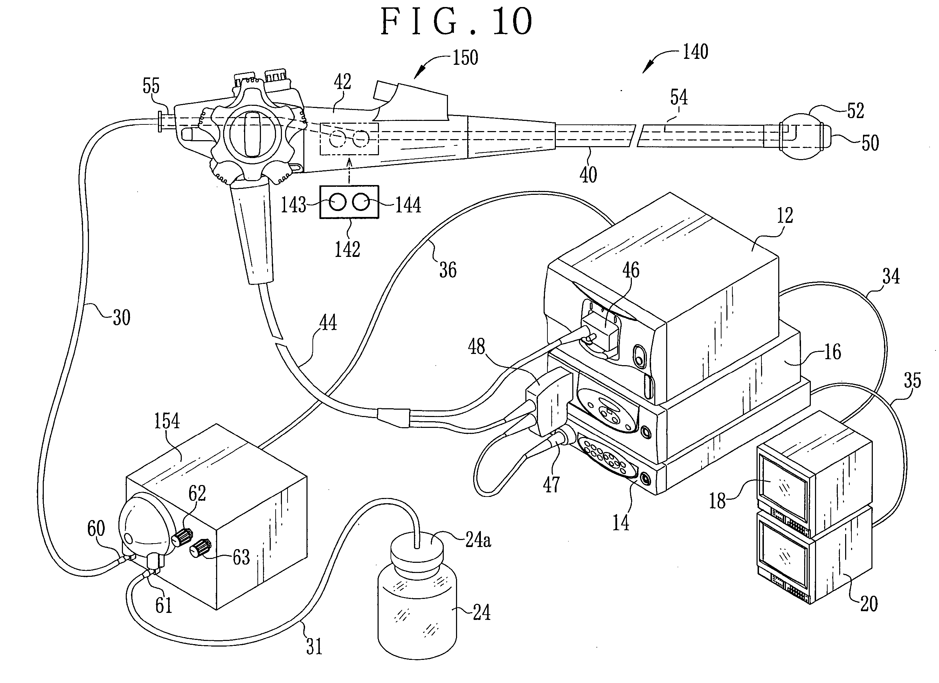

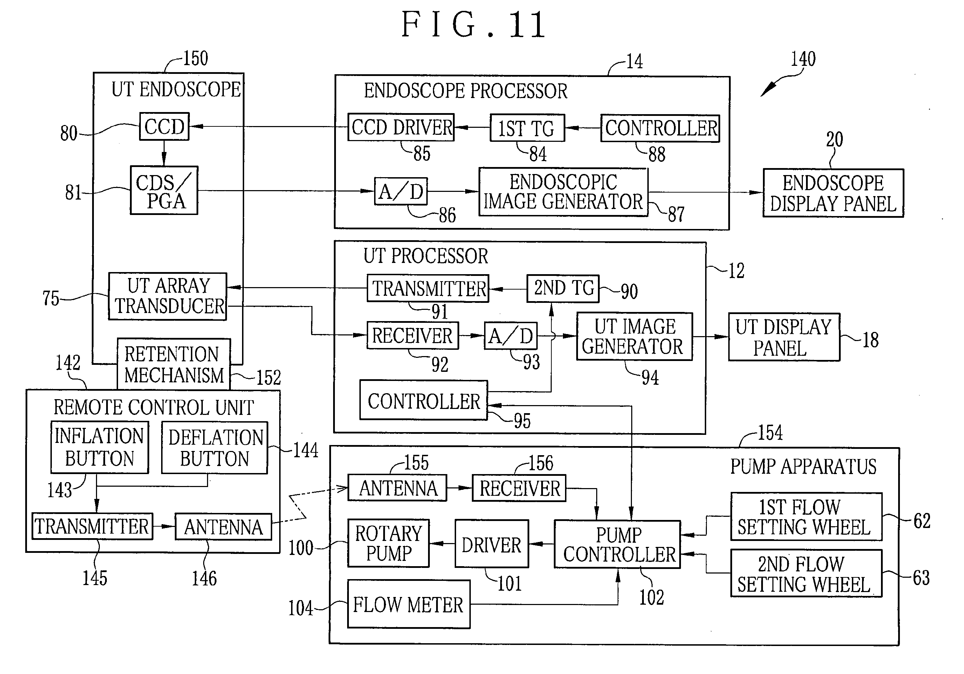

Another preferred ultrasonic diagnosis system 140 is described now. In Fig. 10, a remote control unit 142 has an inflation button 143 and a deflation button 144 as input devices. A retention mechanism 152 is disposed on an ultrasonic endoscope 150, and retains the remote control unit 142 on the handle 42 in a removable manner. See Fig. 11. Examples of the retention mechanism 152 include a sliding type such as an accessory shoe of a camera, a rotatable type such as a bayonet mechanism, a screw type, a magnet type or the like well-known in the art.

In Fig. 11, a transmitter 145 is incorporated in the remote control unit 142. An antenna 146 for transmission is connected with the transmitter 145 as well as the inflation and deflation buttons 143 and 144. When the inflation button 143 is depressed, the transmitter 145 generates the inflation signal, and converts this into a signal for wireless communication. A radio wave of the signal is emitted by the antenna 146. When the deflation button 144 is depressed, the transmitter 145 generates the deflation signal, and converts this into a signal for wireless communication. A radio wave of the signal is emitted by the antenna 146.

A pump apparatus 154 includes an antenna 155 and a receiver 156. The antenna 155 receives a radio wave from the remote control unit 142, and converts this into a signal which is input to the receiver 156. The receiver 156 demodulates the signal to obtain the inflation or deflation signal in an initial form, which is input to the pump controller 102. The pump controller 102 controls the rotary pump 100 according to the inflation or deflation signal, to supply or discharge water in relation to the balloon 52.

The inflation and deflation buttons 143 and 144 on the remote control unit 142 are effective in raising the working efficiency of the ultrasonic endoscope 150, because the remote control unit 142 is removable on the handle 42 and sends signals to the pump apparatus 154 wirelessly. Note that the remote control unit 142, although removable in the embodiment, may be disposed on the handle 42 in a stationary manner. Furthermore, it is possible to incorporate the receiver 156 in the ultrasonic processor 12 or the endoscope processor 14, receive signals, and transmit the signals to the pump apparatus 154.

In the above embodiments, the ultrasonic transducer 75 is oriented in a lateral direction in the head assembly of the ultrasonic endoscope 10. However, the ultrasonic transducer 75 of the ultrasonic endoscope 10 can be oriented straight from the distal end of the head assembly, namely in a longitudinal direction. In the above embodiments, the balloon 52 has the two open ends having the ring portions 52a and 52b. However, the balloon 52 in the invention can have a bag shape, and has one closed end positioned opposite to a single open end.

In the above embodiments, the device in connection with the pump apparatus of the invention is the ultrasonic endoscope. However, a device in connection with a pump apparatus of the invention may be an ultrasonic probe for use by insertion through a forceps channel of an electronic endoscope. Also, ultrasonic transmission medium for use in the balloon 52, in place of the water of the embodiments, may be other liquid or gas for the purpose of preventing attenuation of ultrasonic waves. Furthermore, a pump in the pump apparatus, in place of the rotary pump 100, may be any type of pump for the purpose of supply and discharge of transmission medium of transmission.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. An ultrasonic diagnosis system having an ultrasonic endoscope (10, 122, 150) and a pump apparatus (22, 112, 117, 154);
said ultrasonic endoscope including:
an ultrasonic transducer (75) for emitting ultrasonic waves and receiving reflected ultrasonic waves;
a balloon support (70) for supporting a resilient balloon (52) mounted thereon hermetically and removably to cover said ultrasonic transducer;
a first flow opening (55) for passing an ultrasonic transmission medium (24);
one flow channel (54), formed between said first flow opening and said balloon support, for flow of said transmission medium into and out of said balloon in forward and backward directions; an inflation input device (56, 124, 143) for inputting a signal to said pump apparatus for inflating said balloon;
a deflation input device (57, 125, 144) for inputting a signal to said pump apparatus for deflating said balloon;
said pump apparatus including:
a second flow opening (60) connected with said first flow opening by a tube (30);
a pump (100) for supplying said transmission medium through said tube and said flow channel to inflate said balloon, and for discharging said transmission medium from said balloon to deflate said balloon;
a pump controller (102) for control of said pump in response to said inflation signal to supply said transmission medium, and for control of said pump in response to said deflation signal to discharge said transmission medium.

2. An ultrasonic diagnosis system as defined in claim 1, further comprising a processor, connected with said ultrasonic endoscope and said pump apparatus, for inputting said inflation and deflation signals to said pump apparatus.

3. An ultrasonic diagnosis system as defined in claim 1, wherein said ultrasonic endoscope includes plural switches adapted to selected functions, including a first switch for constituting said inflation input device and a second switch for constituting said deflation input device.

4. An ultrasonic diagnosis system as defined in claim 1, wherein said ultrasonic endoscope includes a transmitter, having said inflation and deflation input devices, for wirelessly transmitting said inflation and deflation signals.

5. An ultrasonic diagnosis system as defined in claim 4, further comprising a retention mechanism for retaining said transmitter on said ultrasonic endoscope removably.

6. An ultrasonic diagnosis system as defined in claim 1, wherein said pump apparatus further includes:
a first flow setting device for setting a supply amount of said transmission medium;
a second flow setting device for setting a discharge amount of said transmission medium;
wherein said pump controller controls said pump to supply said transmission medium at said supply amount set by said first flow setting device and to discharge said transmission medium at said discharge amount set by said second flow setting device.

7. An ultrasonic diagnosis system as defined in claim 1, wherein said pump apparatus further includes a measuring device for measuring at least one of a supply amount of said transmission medium to said balloon and a discharge amount of said transmission medium from said balloon.

8. An ultrasonic diagnosis system as defined in claim 7, further comprising:
an evaluator for checking propriety of said supply amount or said discharge amount from said measuring device by comparison with a reference amount; and
an alarm unit for generating an alarm signal if said supply amount or said discharge amount is found higher than said reference amount by said evaluator.

9. An ultrasonic diagnosis system as defined in claim 7, wherein said measuring device is a flow meter.

10. An ultrasonic diagnosis system as defined in claim 7, wherein said measuring device includes a timer for measuring at least one of a supply time and discharge time of said transmission medium with said pump.

11. An ultrasonic diagnosis system as defined in claim 7, wherein said pump is a rotary pump for supplying and discharging said transmission medium by rotations in rotor control;
said measuring device detects at least one of a number and angle of said rotations of said rotor control.

12. A pump apparatus for connection with an ultrasonic endoscope (10, 122, 150);
said ultrasonic endoscope including an ultrasonic transducer (75) for emitting ultrasonic waves and receiving reflected ultrasonic waves, a balloon support (70) for supporting a resilient balloon (52) mounted thereon hermetically and removably to cover said ultrasonic transducer, a first flow opening (55) for passing an ultrasonic transmission medium (24), one flow channel (54), formed between said first flow opening and said balloon support, for flow of said transmission medium into and out of said balloon in forward and backward directions, an inflation input device (56, 124, 143) for generating a signal for inflating said balloon, a deflation input device (57, 125, 144) for generating a signal for deflating said balloon, said pump apparatus comprising:
a second flow opening (60) connected with said first flow opening by a tube (30);
a pump (100) for supplying said transmission medium through said tube and said flow channel to inflate said balloon, and for discharging said transmission medium from said balloon to deflate said balloon;
a pump controller (102) for control of said pump in response to said inflation signal to supply said transmission medium, and for control of said pump in response to said deflation signal to discharge said transmission medium.

13. A pump apparatus as defined in claim 12, further comprising:
a first flow setting device for setting a supply amount of said transmission medium;
a second flow setting device for setting a discharge amount of said transmission medium;
wherein said pump controller controls said pump to supply said transmission medium at said supply amount set by said first flow setting device and to discharge said transmission medium at said discharge amount set by said second flow setting device.

14. A pump apparatus as defined in claim 12, further comprising a measuring device for measuring at least one of a supply amount of said transmission medium to said balloon and a discharge amount of said transmission medium from said balloon.

15. A pump apparatus as defined in claim 14, further comprising:
an evaluator for checking propriety of said supply amount or said discharge amount from said measuring device by comparison with a reference amount; and
an alarm unit for generating an alarm signal if said supply amount or said discharge amount is found higher than said reference amount by said evaluator.
